**Europäisches Patentamt**

**European Patent Office**

**Office européen des brevets**

(11) Veröffentlichungsnummer: **0 446 403 A1**

## (12) EUROPÄISCHE PATENTANMELDUNG

(21) Anmeldenummer: **90109027.4**

(22) Anmeldetag: **14.05.90**

(51) Int. Cl.5: **A61M 5/00, A61M 5/315**

(30) Priorität: **14.03.90 DE 9003072 U**

(43) Veröffentlichungstag der Anmeldung:
**18.09.91 Patentblatt 91/38**

(84) Benannte Vertragsstaaten:
**CH DE FR LI**

(71) Anmelder: **Nothdurft, Klaus**
**Vaihinger Strasse 50**
**W-7000 Stuttgart 80(DE)**

(72) Erfinder: **Nothdurft, Klaus**
**Vaihinger Strasse 50**
**W-7000 Stuttgart 80(DE)**

(74) Vertreter: **Schmid, Berthold et al**
**Patentanwälte Dipl.-Ing. B. Schmid Dr. Ing. G.**
**Birn Falbenhennenstrasse 17**
**W-7000 Stuttgart 1(DE)**

(54) **Gerät zum Einstellen der Füllmenge einer Injektionsspritze.**

(57) Damit ein Sehbehinderter, der sich häufig selbst spritzen muß, eine Einmalspritze (4) mit der vorgeschriebenen Menge des Spritzmittels schnell, genau und sicher füllen kann, wird die Einmalspritze in ein Gerät eingelegt, dessen wesentliche Teile zwei mittels einer Gewindespindel (1) verbundene Halteglieder (2, 3) sowie eine in der hohlen Gewindespindel längsverschiebbares Zug- und Schubelement (7) mit radial über einen Längsschlitz (5) der Gewindespindel (1) vorstehendem Anschlagelement (6) sind. Es wirkt mit einem, einen verstellbaren Anschlag bildenden, auf der Gewindespindel (1) verschraubbaren Schraubglied (22) zusammen, welches die Verschiebebewegung des Anschlagelements (6) und damit auch des mit Hilfe des Zug- und Schubglieds (7) über die Kolbenstange (9) verschobenen Kolbens der Spritze (4) begrenzt.

Fig. 2

Die Erfindung bezieht sich auf ein Gerät zur Einstellung der Füllmenge einer Injektionsspritze, insbesondere einer Einmalspritze mit einem Außenbund oder einem zweiseitigen Grifflappen am rückwärtigen Ende des einen Kolben mit Kolbenstange aufnehmenden Spritzenzylinders mit Füllskala. Injektionsspritzen werden in aller Regel nicht vollständig mit dem Spritzmittel gefüllt, sondern nur teilweise. Wieviel Spritzmittel jeweils aus einer Ampulle od.dgl. herausgesaugt werden muß, richtet sich nach der ärztlichen Verordnung. Bei Patienten mit gutem Augenlicht ist es kein Problem, die Spritze bis zur vorgegebenen Skalenmarke zu füllen. Sehbehinderte haben aber schon weit größere Schwierigkeiten und Blinde können diesen Arbeitsgang überhaupt nicht ausführen. Sie sind in jedem Falle auf die Hilfe einer weiteren Person angewiesen.

Es liegt infolgedessen die Aufgabe vor, ein Gerät der eingangs genannten Art zu schaffen, welches ein genaues Füllen einer Spritze bis zu einem bestimmten Skalenpunkt auch dann möglich macht, wenn der Patient die Skala nicht oder nur mit großer Anstrengung ablesen kann.

Zur Lösung dieser Aufgabe wird erfindungsgemäß vorgeschlagen, daß das Gerät gemäß dem Oberbegriff des Anspruchs 1 entsprechend dem kennzeichnenden Teil dieses Anspruchs ausgebildet ist. In das erfindungsgemäße Gerät wird die Spritze, insbesondere eine Einmalspritze, passender Größe so eingelegt, daß der nadelseitigen Endstellung des Schraubglieds und Anschlagelements die Entleerstellung der Spritze zugeordnet ist. Dies ist gewissermaßen die Nullstellung des Geräts. Wenn man die Steigung der Spindel so wählt, daß ein oder mehrere Spindelgänge einer Skaleneinheit bzw. insgesamt einer Skaleneinheit entsprechen, so kann man durch einfaches Abzählen der vorgenommenen Umdrehungen des Schraubglieds dieses in eine Position an der Schraubspindel bringen, welche einem vorbestimmten Skalenabschnitt zugeordnet ist. Das Schraubglied bildet ein verstellbares Anschlagglied. Wenn man nunmehr ausgehend von der Nullstellung das Zug- und Schubglied so verschiebt, daß sich dessen Anschlagelement gegen das als Anschlagglied wirkende Schraubglied bewegt, so hört diese Verschiebebewegung in dem Moment auf, in dem das Anschlagelement am Schraubglied aufgetroffen ist. Weil bei dieser Verschiebebewegung des Zug- und Schubglieds zugleich auch die Kolbenstange mit dem Kolben im Sinne eines Füllhubs verschoben wird, ist die Spritze beim Auftreffen des Anschlagelements am Schraubglied genau um das vorgesehene Maß gefüllt.

Man kann das Gerät so klein und handlich bauen, daß man die Spritze beim Spritzen des aufgezogenen Mittels am Gerät belassen kann. Aus dem Vorstehenden ergibt sich, daß die Kolbenstange mit dem Mitnehmer des Zug- und Schubglieds verschiebefest verbunden sein muß. Die schiebefeste Verbindung muß zumindest in Füllrichtung des Kolbens bzw. der Kolbenstange gewährleistet sein. Ist sie lediglich in Füllrichtung gewährleistet, so kann man den Kolben am Ende des Füllvorgangs in Füllrichtung noch weiterbewegen, um dadurch eine noch größere Menge ansaugen zu können. Wenn man nunmehr den Mitnehmer in seiner herausgezogenen Position gegenüber den Haltegliedern festhält und anschließend Kolben und Kolbenstange in Spritzrichtung verschiebt, so kann man auf diese Weise eventuell angesaugte Luft vor dem eigentlichen Benutzen der Spritze ausstoßen. Zugleich tritt dann auch die überschüssige Menge des Spritzguts in der bei normaler Benutzung einer Einmalspritze üblichen Weise über die Nadel aus. Dieser Ausspritzvorgang ist beendet, wenn die Kolbenstange wieder die Position eingenommen hat, die sie am Ende des eigentlichen Füllvorgangs inne hatte. Man kann nun die Kolbenstange zusammen mit dem Mitnehmer in die Ausgangslage zurückschieben und dabei dem zu behandelnden Körper das Spritzgut zuführen.

Dieses Gerät ist aber nicht nur für Sehschwache, Sehbehinderte oder Blinde geeignet, vielmehr erlaubt es auch bei unveränderter Einstellung, die einer ständig gleichen Spritzmenge entspricht, das rasche und genaue Füllen auch für einen Patienten ohne Sehbehinderung. Er erspart sich auf diese Weise das Ablesen der Skala.

Eine Weiterbildung der Erfindung sieht vor, daß das Schraubglied ein exzentrisches Gewinde oder ein radial vorstehendes Element, insbesondere eine Längsleiste od.dgl. hat. Im ersten Falle kann man in Verbindung mit einem beispielsweise nockenförmigen Querschnitt und im zweiten Falle mit Hilfe der Längsleiste die einzelnen Umdrehungen leicht ermitteln. Gleiches wäre selbstverständlich auch mit einer Rille, einem Farbauftrag od.dgl. möglich, jedoch ist letzterer für Sehbehinderte keine brauchbare Lösung.

Eine andere Variante der Erfindung ist durch eine radial einwärts vorstehende federelastische Zunge od.dgl. des Schraubglieds gekennzeichnet, die mit dem Längsschlitz der Gewindespindel ratschenartig zusammenwirkt. Die Zunge sollte breiter sein als die Längsschlitzbreite und sie darf bei Übereinstimmung mit dem Längsschlitz in letztere nicht einrasten. Andererseits soll aber jede einzelne Umdrehung bei Übereinstimmung von Zunge und Längsschlitz sicher zu fühlen und/oder zu hören sein. Das Drehen muß in beiden Drehrichtungen trotzdem leicht möglich sein.

Eine weitere Ausgestaltung der Erfindung besteht darin, daß die beiden Halteglieder als etwa U-förmige Halteböcke ausgebildet sind, mit quer zur Längsachse der Gewindespindel gerichteten U-Schenkeln. Sie ermöglichen ein rasches Ansetzen und Abnehmen der Spritze. Die lichte Weite zwischen den U-Schenkeln muß dem festzuhaltenden Querschnitt der Spritze entsprechend gewählt werden.

Bei einer besonders bevorzugten Ausführungsform der Erfindung weist der Mitnehmer eine etwa U-förmige Gestalt auf, die derjenigen der Halteglieder ähnlich sein kann. Soweit erforderlich, also insbesondere bei einer über die gesamte Länge gleichdicken Kolbenstange, muß letztere klemmend am Mitnehmer gehalten werden. In diesem Falle entsteht dann eine zug- und druckfeste, aber bei entsprechender Wahl der Toleranzen trotzdem gut lösbare Ankupplung.

Eine andere Ausbildung des Geräts mit am freien Kolbenstangenende angebrachtem Drücker ist dadurch gekennzeichnet, daß sich der Mitnehmer zwischen dem Drücker und dem grifflappenseitigen Halteglied befindet, wobei die Kolbenstange längsverschiebbar im Mitnehmer gelagert ist. Hier wird also auf eine klemmende Verbindung von Kolbenstange und Mitnehmer verzichtet. Dafür ist die Verbindung auch nur in einer einzigen Richtung, nämlich beim Saughub des Kolbens, gewährleistet. Beim Spritzen muß die Kraft auf den Drücker der Spritze aufgegeben werden, die dann ihrerseits den Mitnehmer samt Zug- und Schubglied in die Nullstellung zurückschiebt. Dafür hat diese Ausbildung den Vorteil, daß man einen Überhub mit entsprechendem Ausspritzen eventuell angesaugter Luft machen kann.

Eine weitere bevorzugte Variante der Erfindung kennzeichnet sich dadurch, daß wenigstens eines der Halteglieder, insbesondere das nadelseitige, als Klemmhalteglied ausgebildet ist. Üblicherweise verwendete Einmalspritzen haben nicht nur am hinteren Endbereich zwei Grifflappen und am freien Ende der Kolbenstange einen Drücker, sondern auch am nadelseitigen Endbereich einen kleinen Außenbund oder einen Absatz. Dieser hat vom nadelseitigen Ende einen gewissen Abstand. Diesen Teil des Zylinders kann man am nadelseitigen Halteglied klemmend festhalten. Gegebenenfalls kann man auch den gegenüberliegenden Endbereich des Zylinders vom Grifflappen bis zum drückerseitigen Ende des Zylinders in gleicher Weise durch das andere Halteglied klemmend festhalten. Dieser Durchmesser ist in der Regel größer, weswegen die U-Schenkel dieses Halteglieds einen größeren Seitenabstand aufweisen.

Die Erfindung wird nachstehend anhand der Zeichnung näher erläutert. Die Zeichnung zeigt ein Ausführungsbeispiel der Erfindung. Hierbei stellen dar:

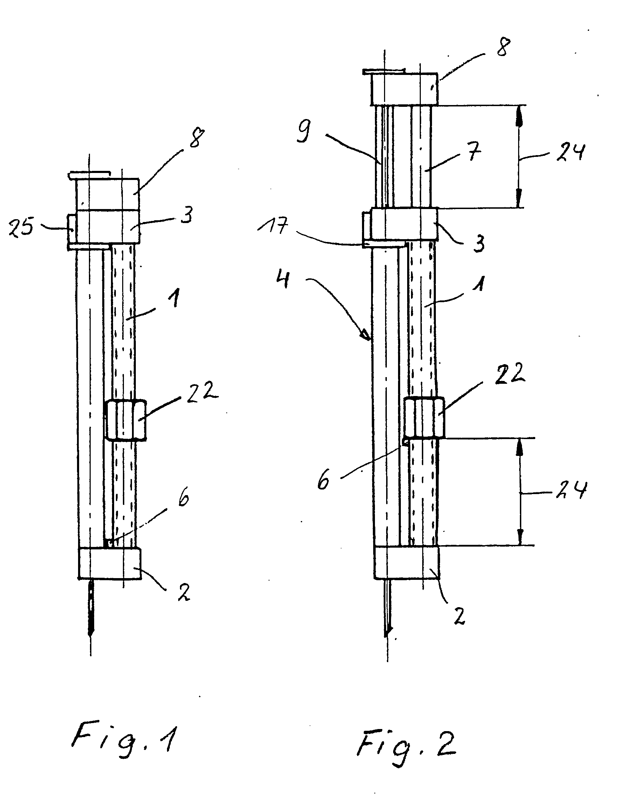

Fig. 1     Eine Seitenansicht des Geräts mit eingelegter Einmalspritze bei einer gewählten Einstellung des Schraubglieds, mit dem Kolben in der Ausgangsstellung,

Fig. 2     eine entsprechende Darstellung am Ende des Füllhubs,

Fig. 3     in vergrößertem Maßstab und teilweise längsgeschnitten eine Darstellung gemäß Fig. 1,

Fig. 4     eine Draufsicht auf Fig. 3,

Fig. 5     eine um 90° gedrehte Darstellung der Fig. 3,

Fig. 6     ein Schnitt gemäß der Linie VI-VI der Fig. 5.

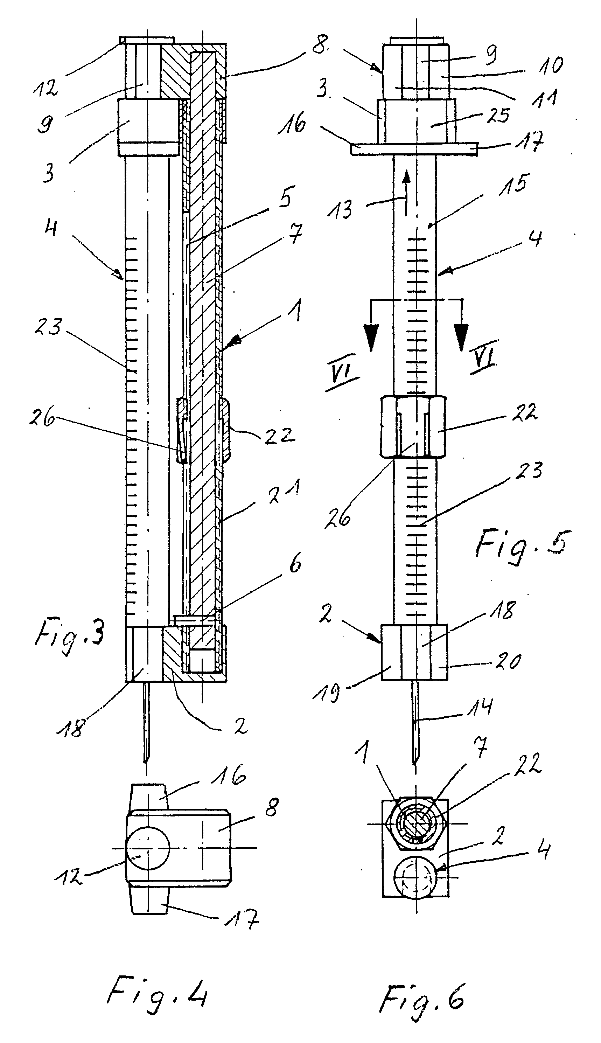

Eine Gewindespindel 1 verbindet die beiden Halteglieder 2 und 3 miteinander. Letztere dienen zum Festhalten einer Einmalspritze 4. Die Gewindespindel ist mit einem Längsschlitz 5 ausgestattet, die von einem radial vorstehenden Anschlagelement 6 eines Zug- und Schubglieds 7 nach außen hin durchsetzt wird. Letzteres trägt an seinem freien, über die Gewindespindel 1 axial vorstehenden Ende einen Mitnehmer 8 für die Kolbenstange 9 der Spritze 4. Er besitzt gemäß Fign. 3 und 5 eine im wesentlichen U-förmige Gestalt, wobei die Kolbenstange zwischen die beiden U-Schenkel 10 und 11 greift. Beim Ausführungsbeispiel befindet sich am freien Ende der Kolbenstange 9 ein Drücker 12, weswegen die Kolbenstange von den beiden U-Schenkeln nicht klemmend gehalten werden muß. Es ist insbesondere vorgesehen, daß man die Kolbenstange relativ zu den U-Schenkeln 10 und 11 im Sinne des Pfeils 13 verschieben kann.

Am von der Nadel 14 entfernten Ende des Spritzen-Zylinders 15 sind an diesem zwei, vorzugsweise miteinander verbundene Grifflappen 16 und 17 angeformt. Sie liegen an der Innenfläche des Halteglieds 3 an.

Am nadelseitigen Ende befindet sich entweder ein dementsprechender Außenbund oder eine Querschnittsreduzierung des Zylinders 15. Der im Querschnitt dünnere Bereich 18 des Zylinders 15 stützt sich an der Innenfläche des anderen Halteglieds 2 ab. Die Einmalspritze 4 ist auf diese Weise in axialer Richtung verschiebefest am Gerät gehalten.

Auch die beiden Halteglieder 2 und 3 haben eine im wesentlichen U-förmige Gestalt. Dabei bilden zumindest die beiden U-Schenkel 19 und 20 eines Halteglieds, vorzugsweise des Halteglieds 2, Klemmbacken für den eingreifenden Bereich 18 des Spritzen-Zylinders 15. Die U-Schenkel des Halteglieds 3 können in gleicher Weise Klemmbacken bilden.

Auf dem Bolzengewinde 21 der Gewindespindel 1 befindet sich ein mutterartiges Schraubglied 22. Zwischen diesem und dem spritzenseitigen Halteglied 2 befindet sich das Anschlagelement 6.

Es nimmt in Fig. 3 seine Ausgangsstellung ein, der die nadelseitige Endstellung des nicht gezeichneten Kolbens der Einmalspritze 4 zugeordnet ist.

Ausgehend von dieser Null- oder Ausgangsstellung des Anschlagelements 6 ist dessen Verschiebebewegung bis zum Auftreffen am Schraubglied 22 möglich, weswegen letzteres einen verstellbaren Anschlag für das Anschlagelement 6 bildet.

Der Zylinder der Einmalspritze 4 ist mit einer Skala 23 versehen. Der Abstand benachbarter Skalenstriche entspricht vorzugsweise der Steigung eines Gewindeganges des Bolzengewindes 21. Auf diese Weise ist eine einfache Zuordnung eines bestimmten Skalenabschnitts bzw. einer bestimmten Füllmenge der Spritze zu einer entsprechenden Umdrehungszahl des Schraubglieds 22, ausgehend vom Halteglied 2, möglich. Dem jeweils eingestellten Verschiebeweg 24 des Anschlagelements 6 entspricht der gleiche Verschiebeweg der Kolbenstange 9 mit dem nadelseitig angebrachten Kolben der Spritze. Es bleibt noch nachzutragen, daß das grifflappenseitige Ende des Spritzen-Zylinders 15 mit 25 bezeichnet ist.

Damit man die einzelnen Umdrehungen leicht fühlen und gegebenenfalls auch hören kann, ist am Schraubglied 22 eine federelastische Zunge 26 angebracht, insbesondere angeformt. Sie wirkt mit dem Längsschlitz 5 der hohlen Gewindespindel 1 in der Art einer Ratscheinrichtung zusammen. Jedes Mal, wenn diese Zunge am Längsschlitz 5 angekommen ist, rastet sie darin etwas ein, jedoch nur so weit, daß sie genauso leicht wieder austreten wie eintreten kann.

## Patentansprüche

1. Gerät zur Einstellung der Füllmenge einer Injektionsspritze, insbesondere einer Einmalspritze (1) mit einem Außenbund oder einem zweiseitigen Grifflappen (16, 17) am rückwärtigen Ende des einen Kolben mit Kolbenstange (9) aufnehmenden Spritzen-Zylinders (15) mit Füllskala (23), gekennzeichnet durch zwei über eine hohle, längsgeschitzte Gewindespindel (1) miteinander verbundene Halteglieder (2, 3) für das nadelseitige Ende (18) des Spritzen-Zylinders (15) einerseits und das grifflappenseitige Ende (25) andererseits, sowie ein Schraubglied (22) an der Gewindespindel (1) und ein in letzterer verschiebbares Zug- und Schubglied (7) mit radial vorstehendem Anschlagelement (6), das sich zwischen dem nadelseitigen Halteglied (2) und dem Schraubglied (22) befindet, wobei das Zug- und Schubglied (7) an seinem freien Ende einen Mitnehmer (8) für die Kolbenstange (9) trägt und wobei außerdem der nadelseitigen Endstellung von Schraubglied (22) und Anschlagelement (6) die Entleerstellung der Spritze (4) zugeordnet ist und einer Skaleneinheit der Spritze (4) eine Umdrehung oder ein ganzes Vielfaches einer Umdrehung des Schraubglieds (22) zugeordnet ist.

2. Gerät nach Anspruch 1, dadurch gekennzeichnet, daß das Schraubglied (22) ein exzentrisches Gewinde oder ein radial vorstehendes Element, insbesondere eine Längsleiste od.dgl., aufweist.

3. Gerät nach Anspruch 1, gekennzeichnet durch eine radial einwärts vorstehende federelastische Zunge (26) od.dgl. des Schraubglieds (22), die mit dem Längsschlitz (5) der Gewindespindel (1) ratschenartig zusammenwirkt.

4. Gerät nach wenigstens einem der vorhergehenden Ansprüche, dadurch gekennzeichnet, daß die beiden Halteglieder (2, 3) als etwa U-förmige Halteböcke ausgebildet sind, mit quer zur Längsachse der Gewindespindel (1) gerichteten U-Schenkel (19, 20).

5. Gerät nach wenigstens einem der vorhergehenden Ansprüche, dadurch gekennzeichnet, daß der Mitnehmer (8) eine etwa U-förmige Gestalt aufweist.

6. Gerät nach Anspruch 5, mit am freien Kolbenstangenende angebrachtem Drücker (12), dadurch gekennzeichnet, daß sich der Mitnehmer (8) zwischen dem Drücker (12) und dem grifflappenseitigen Halteglied (3) befindet, wobei die Kolbenstange (9) längsverschiebbar im Mitnehmer (8) gelagert ist.

7. Gerät nach wenigstens einem der vorhergehenden Ansprüche, dadurch gekennzeichnet, daß wenigstens eines der Halteglieder (2, 3), insbesondere das nadelseitige, als Klemmhalteglied ausgebildet ist.

Fig. 1

Fig. 2

Fig.3

Fig.4

Fig.5

Fig.6

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | US - A - 2 739 589 (D.E. YOCHEM) <br> * Spalte 3, Zeilen 23ff; Fig. 3,5 * <br> * Spalte 2, Zeilen 32ff; Fig. 1,2,4 * | 1,5,6 <br><br><br> 4,7 | A 61 M 5/00 <br> A 61 M 5/315 |
| A | US - A - 3 965 945 (ROSS) <br> * Spalte 3, Zeilen 30ff; Fig. 5 * | 2 | |
| A | US - A - 4 810 249 (HABER et al.) <br> * Spalte 7, Zeilen 1ff; Fig. 10,11 * | 3 | |

RECHERCHIERTE
SACHGEBIETE (Int Cl⁵)

A 61 M 5/00

Der vorliegende Recherchenbericht wurde für alle Patentansprüche erstellt.

| Recherchenort | Abschlußdatum der Recherche | Prüfer |
|---|---|---|
| WIEN | 11-12-1990 | SCHNEEMANN |